# EUROPEAN PATENT SPECIFICATION

(11) **EP 1 471 717 B1**
(45) Date of publication and mention of the grant of the patent: **28.02.2007**
(21) Application number: 04076968.9
(22) Date of filing: 14.05.2002
(51) Int. Cl.: H04M 1/02, H04M 1/725, H04B 5/00

(54) **A portable communication device**
Ein tragbares Kommunikationsgerät
Un appareil de communication portable

(30) Priority: 14.05.2001 GB 0111722; 10.12.2001 GB 0129491; 10.12.2001 GB 0129490; 10.04.2002 GB 0208275
(43) Date of publication of application: 27.10.2004
(62) Divisional of application: 02769514.7
(73) Proprietor: Innovision Research & Technology PLC, Cirencester, Gloucestershire GL7 1RQ (GB)
(72) Inventor: White, Andrew, Innovision Research & Tech. PLC, Wokingham Berkshire RG40 1XS (GB); Borrett, Marc a., Innovision Research & Tech. PLC, Wokingham Berkshire RG40 1XS (GB); Mapleston, David Bernard, Wokingham Berkshire RG40 1XS (GB)
(74) Representative: Clark, Jane Anne

(56) References cited:
- EP-A- 0 820 178
- WO-A-00/79771
- WO-A-98/58509
- WO-A-99/60713
- GB-A- 2 327 565
- US-A- 5 553 314
- US-A- 5 796 351

## Description

This invention relates to cellular telephone devices such as cellular telephones arranged to operate using a mobile telecommunications network.

Telephones that operate using a mobile telecommunications network are variously known as mobile telephones, cellular telephones and cellphones. For simplicity, the term "cellular telephone" will be used hereinafter.

GB-A-2, 327, 565 describes a system in which user terminals are provided for use within a specific single environment (such as a shop, museum or public transport stop or station) having a base station for communicating with those user terminals. In this system, an item for sale in the shop (or on display in the museum or the stop or station) has a label. The labels each carry a machine-readable recording of a URL of a web page which can be read in a wireless manner by the dedicated user terminals. The labels may be tagging devices, barcodes, magnetic stripes or any other form of machine-readable label. In the system of GB-A-2,327,565, once a user has been provided with such a user terminal, he or she can use the user terminal to read the labels on the items in the specific environment in which the system operates. When a URL is read by one of the user terminals, that user terminal then communicates the URL via another wireless link to the base station. The base station then communicates with servers via the Internet to retrieve the web page corresponding to the URL communicated to it by the user terminal so that the base station can supply the corresponding web page to the user terminal.

EP-A-0492569 describes a system and method for the non-contact transmission of data wherein a fixed station has an antenna which transmits a first RF signal which is inductively coupled to a portable data carrier which superimposes a second signal generated within the portable data carrier on the first signal.

EP-A-0400764 describes a transaction system enabling a portable token to co-operate with a fixed terminal by being inductively coupled to the fixed terminal. The terminal is a permanent fixture in a building or vehicle and has a fairly large, possibly of the order 15 cm by 15 cm, inductive loop to enable inductive coupling to the portable token.

According to the present invention, there is provided a cellular telephone device having:
a telecommunications radio frequency transceiver for enabling transmission and reception of communications over a mobile telecommunications network;
control means for controlling operation of the cellular telephone device; and
reading means having reader inductive coupling means for, when the cellular telephone device is in close proximity to an article carrying a data storage device having a data store and a data storage device inductive coupling means, inductively coupling a radio frequency signal provided by a radio frequency signal supplying means of the cellular telephone device to the data storage device inductive coupling means to cause the data storage device to modulate that radio frequency signal in accordance with data stored by the data store of the data storage device,
the reading means having data extracting means for extracting the data from the modulated radio frequency signal to affect an operation or service of the cellular telephone device.

In an embodiment, the data may comprise data for at least one of: graphics; cellular telephone device services; subscription services; network services; services; promotions; and advertisement; fascia identification data; games software for games that may be played on the cellular telephone device including a new game or modifications for such games software already installed on the cellular telephone device ; and data for enabling a user to access any one or more of the above types of data using their cellular telephone device, for example an access code to enable access to control data already stored by the cellular telephone device or data such as a telephone number, Internet address or WAP address from which control data can be accessed, or the user's cellular network provider or another third party service provider.

In an embodiment, a data storage device derives a power supply from the radio frequency signal supplied by the cellular telephone device. The use of a data storage device that does not require its own power supply enables costs to be kept down and, moreover, because such passive data storage devices are relatively cheap to produce and can be incorporated into products relatively easily (for example during a plastics moulding process), the incorporation of the passive data storage device does not add significantly to costs.

As used herein the term "passive data storage device" means a device that is not self-powered but that derives power from an inductively coupled radio frequency signal.

In an embodiment, data is communicated by modulation by the data storage device of the same radio frequency signal supplied by the cellular telephone device from which the data storage device derives its powers.

Embodiments of the present invention will now be described, by way of example, with reference to the accompanying drawings, in which:
Figure 1 shows a diagrammatic perspective view of a cellular telephone with a removable fascia embodying the invention separated from a main body of the cellular telephone;
Figure 2 shows a functional block diagram of functional components of a cellular telephone embodying the invention, including a reader unit for enabling detection of a replacement fascia;
Figure 3 shows a functional block diagram of an embodiment of the reader unit shown in Figure 2;
Figure 4 shows a functional block diagram of an example of a passive data storage device;
Figure 5 shows a more detailed functional block diagram of an embodiment of the reader unit;
Figure 6 shows a more detailed functional block diagram of one example of a passive data storage device;
Figure 7 shows a functional block diagram of another example of a passive data storage device;
Figure 8 shows a functional block diagram of another example of a cellular telephone embodying the present invention;
Figure 9 shows a functional block diagram of an example of a passive data storage device for use as a detectable control device shown in Figure 8;
Figures 10 to 12 are very diagrammatic views of examples of user input devices;
Figure 13 shows a block diagram of a cellular telephone not falling within the invention claimed;
Figure 14 shows a block diagram illustrating another example of a cellular telephone embodying the invention; and
Figure 15 shows an example of a vending system.

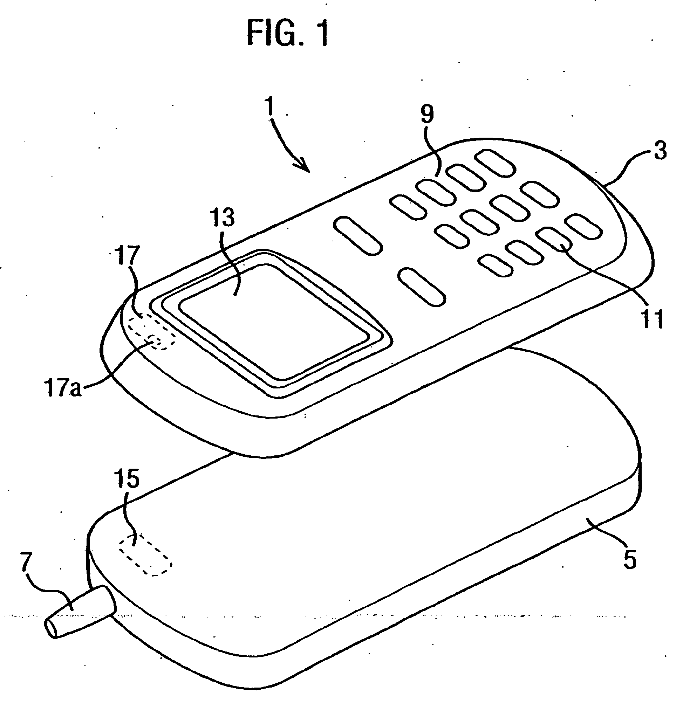

Figure 1 illustrates a typical cellular telephone or cellphone 1 comprising a main body 5 and an attachable, and in this case also removable, fascia 3. The fascia 3 is formed with a user input array 9 which comprises a plurality of holes 11 for engagement with key pads (not shown) provided on the main body 5 of the cellular telephone 1. The fascia 3 is also provided with, in this example, a transparent plastics window 13 through which a liquid crystal display (LCD) (not shown in Figure 1) provided on the main body 5 of the cellular telephone 1 can be viewed. The cellular telephone 1 also comprises an aerial 7 which allows telecommunication signals to be transmitted and received.

Mounted upon, or embedded within, an inside surface of the removable fascia 3 is a passive data storage device 17. Generally, the fascia 3 will be moulded from a plastics material and the passive data storage device 17 encapsulated in a housing fitted to (for example embedded in or mounted to the surface of) the fascia 3 during the moulding process.

The main body 5 of the cellular telephone 1 carries a reader unit 15. The reader unit 15 is positioned such that, when the fascia 3 is fitted to the main body 5, the passive data storage device 17 will be in range of, as shown will lie adjacent to, the reader unit 15 so that, as will be described in detail below, couplers of the passive data storage device 17 and reader unit 15 couple to enable the passive data storage device 17 to derive a power supply from a signal supplied by the reader unit 15 and, when so activated, to transmit control data contained in its memory.

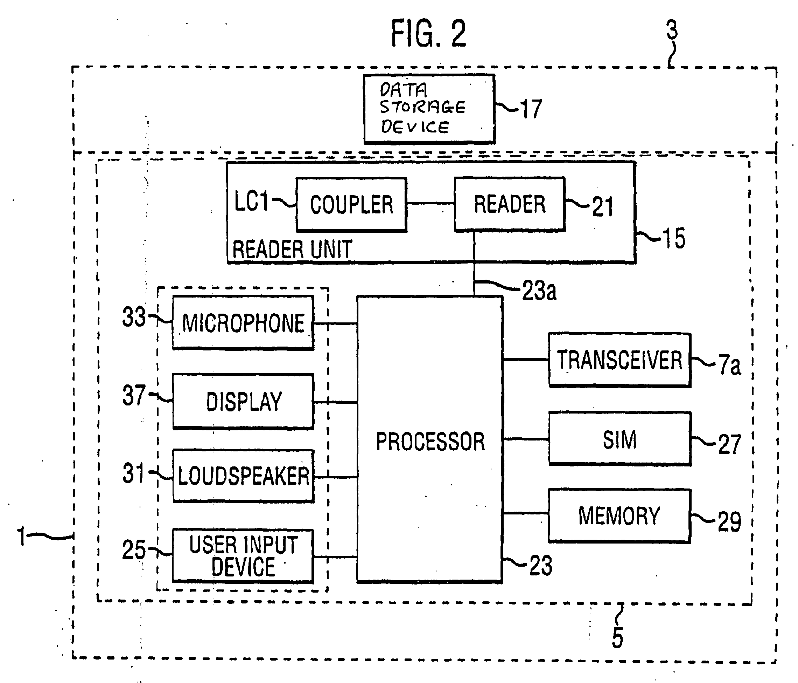

Figure 2 shows a functional block diagram of the cellular telephone 1. The main body 5 of the cellular telephone 1 comprises: a microphone 33 and a loudspeaker 31 enabling a user to input speech and hear audio output respectively; a user input device 25 (in this case a keyboard) enabling the user to input numbers to be called, other information and instructions for controlling various features of the cellular telephone 1; a display 37 on which incoming or outgoing telephone numbers, SMS text messages and other information can be displayed; and a transceiver 7a which with the aerial 7 shown in Figure 1 enables transmission and reception of communications over a mobile telecommunications network using, for example, the GSM (Global System for Mobile Communications), GPRS (General Packet Radio Service) or 3G (Third Generation, GSM) system, or future such networks.

The above functional components are coupled, via appropriate interfaces (not shown) to a cellular telephone processor 23 which controls the overall operation of the cellular telephone 1. The cellular telephone processor 23 is associated with a non-volatile memory 29 which has a read-only portion which contains control operation software data and information data and a writable portion that, for example, enables storage of telephone numbers and messages input to the cellular telephone by the user or received by the cellular telephone over the mobile telecommunications network.

As is known in the art, the cellular telephone 1 further comprises a subscriber identification module (SIM) 27. The SIM 27 is a detachable module which provides user specific data and also algorithms and data specific to the operator of the mobile telecommunications service provider. The cellular telephone processor 23 is coupled to the reader unit 15 via line 23a.

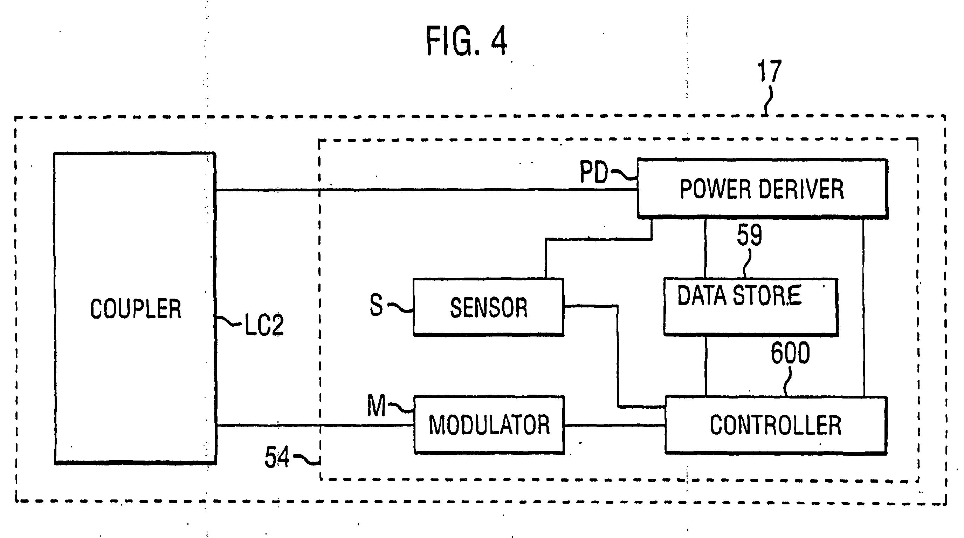

The reader unit 15 comprises a reader 21 which is coupled to the cellular telephone processor 23 via line 23a and which is also coupled to a first coupling element or coupler LC1 arranged to couple to a similar second coupling element LC2 (see Figure 4) carried_by the passive data storage device 17 when the replacement fascia or cover portion 3 is fitted to the main body of the cellular telephone.

Although not shown in Figure 2, it will be appreciated that the components of the cellular telephone 1 and the reader unit 15 will be powered by the battery of the cellular telephone 1, although a separate power supply may be provided.

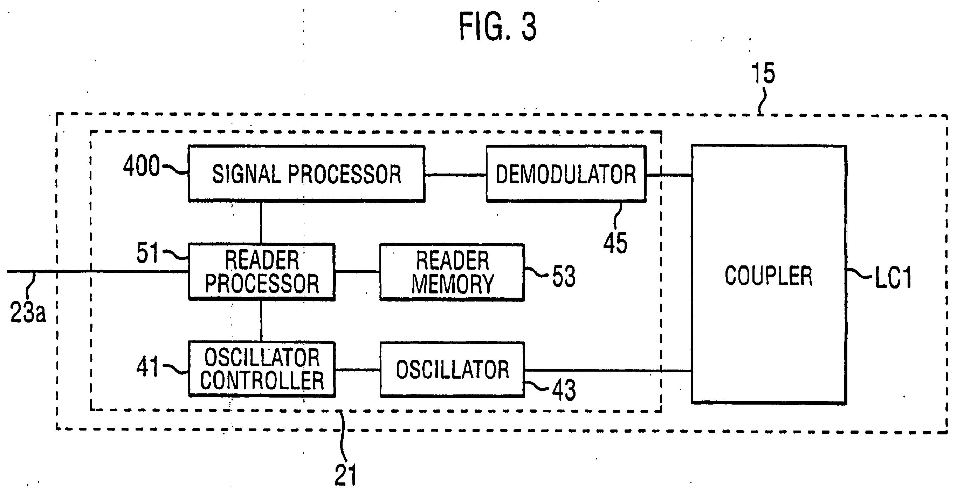

Figure 3 shows an embodiment of the reader unit 15 which consists of the coupling element or coupler LC1 and the reader 21. The reader 21 has a reader microprocessor or microcontroller 51 having a memory 53 which will generally be non-volatile but could be volatile if backed-up, for example battery backed-up. An oscillator 43 controlled by an oscillator controller 41 under the control of the reader microprocessor 51 supplies an oscillator output signal to the coupling element LC1. The reader microprocessor 51 controls the oscillator control 41 to control activation of the oscillator 43 and, in this embodiment, also controls the oscillator control 41 to interrupt the output of the oscillator 43 to provide a signal from which the data storage device 17 within the fascia 3 can derive a clock signal as will be described below.

The first coupling element LC1 is also coupled to a demodulator 45 which enables the modulation to be recovered from an amplitude modulated signal. The recovered modulation is supplied to signal processor 400 which supplies a digital signal representing a string of binary ones and zeros to the reader microprocessor 51. In response to receipt of control data from the passive data storage device, the reader microprocessor 51 communicates with the cellular telephone processor 23 on data line 23a (see Figure 2) so as to affect the functionality or operating characteristics of the cellular telephone.

Figure 4 shows a simplified block diagram of one example of a passive data storage device forming the detectable component 17. As shown, the passive data storage device 17 comprises a passive data storage unit 54 and the coupler or coupling element LC2. The passive data storage unit 54 consists of a power deriver PD that derives a power supply for the passive data storage unit54 from the oscillator signal coupled from the reader unit 15 (shown in Figure 3) to the passive data storage device 17 by coupling of the first and second coupling element LC1 and LC2, a data store 59 in the form of a non-volatile memory storing control data for supply to the reader unit 15 and a controller 600 for controlling reading of data from the data store 59 and supply of that data to a modulator M that modulates the oscillating signal inductively coupled to the passive data storage device 17. Optionally, the passive data storage device may include a sensor S for reasons that will be set out below.

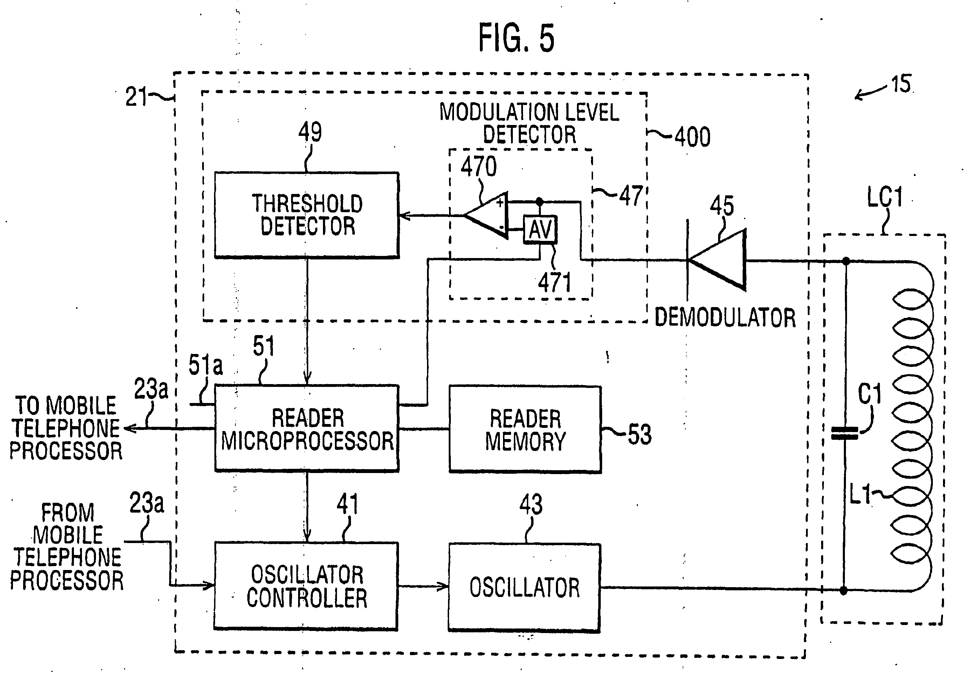

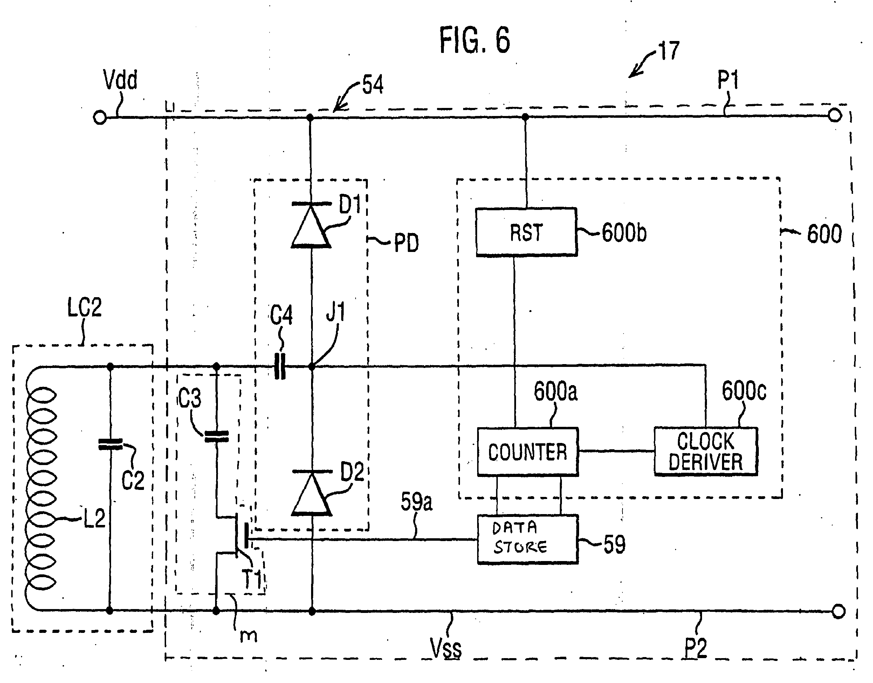

Figures 5 and 6 show, respectively, block diagrams illustrating one example of the reader unit 15 and one example of the passive data storage device 17. The passive data storage device 17 and reader unit 15 are adapted to communicate via inductive coupling and, in this example, each of the couplers LC1 and LC2 consists of a parallel connection of a capacitor and an inductor C1 and L1 and C2 and L2, respectively. The inductive couplers may form a tuned circuit, although this is not necessary at short range. The optional sensor S is not shown in Figure 6.

In the example shown in Figure 5, the demodulator 45 is a simple diode rectifier while the signal processor 400 consists of modulation level and threshold detectors 47 and 49. The modulation level detector 47 comprises a comparator 470 and an averaging circuit 471 controlled by the reader microprocessor 51. The output from the demodulator 45 is supplied to the positive input of the comparator 470. The average of the output of the demodulator 45 is supplied by the averaging circuit 471 to the negative or inverting input of the comparator 470.

The output of the comparator 470 is supplied to the threshold detector 49 which supplies to the reader microprocessor 51 either a one or a zero, depending upon the relationship, between the signal and the threshold.

In this example, the oscillator controller 41 is a logic circuit or a transistor switch which controls the oscillator 43 to switch on or off the carrier signal to the first coupling element LC1 in accordance with a signal received from the reader microprocessor 51 and the averaging circuit 471 generally consists of an averaging capacitor connected between the inverted input of the comparator 470 and ground by a transistor switch or transmission gate which is controlled by the reader microprocessor 51 so as to be conducting while the carrier signal is present and after transients have settled but is off while the carrier is off and during carrier turn-on transients so that averaging is only carried out while there is a steady carrier signal.

As shown in Figure 6, the power deriver PD of the passive data storage unit 54 comprises series-connected diodes D1 and D2 and a capacitor C4 coupled between the coupling element LC2 and a junction J1 between the anode of the diode D1 and a cathode of the diode D2. The cathode of the first diode D1 is connected to a first power supply rail P1 (Vdd) while the anode of the second diode D2 is connected to a second power supply rail P2 (Vss). The capacitor C4 and the diodes D1 and D2 act effectively as a voltage doubler enabling the peak to peak voltage of a received AC or oscillating signal inductively coupled to the passive data storage device 17 by the coupling elements LC1 and LC2 to be used by the diodes D1 and D2 to derive a power supply for the passive data storage device 17 from the oscillating signal.

It will, of course, be appreciated that, in the interests of simplicity, the power supply connections to the remaining components of the passive data storage device 17 are not shown in Figure 6.

In this example, the controller 600 comprises a clock signal deriver 600c in the form of a missing pulse detector which is coupled to the junction J1 to derive a clock signal for the data storage unit 54 from the interrupted oscillator signal supplied by the oscillator 43 and an address counter 600a clocked by the clock signal. The controller 600 may also include a reset switch 600b to reset the counter 600a if the passive data storage device 54 is not powered for a predetermined time.

In this example, the controller 600 causes data to be read out from the data store 59 directly to the modulator M which comprises a series-connection of a FET T1 and a capacitor C3 coupled across the capacitor C2 of the coupler LC2. Thus, an output 59a of the data store 59 is coupled to the gate of the FET T1. In this example, the data store 59 is a serial read-only memory (ROM). It may, however, be any form of non-volatile memory that does not require battery backup such as a ROM, an EE-PROM (electrically erasable programmable ROM), a flash memory, F-RAM and so on.

When, as shown in Figure 1, the replaceable fascia 3 is fitted to the main body 5 of the cellular telephone 1 the coupling element LC1 of the subsidiary unit 17 lies adjacent and in close proximity to the coupling element LC2 of the reader unit 15, inductively coupling the passive data storage device 17 to the reader unit 15. The oscillator 43 of the reader 21 generates a high or RF (radio frequency), typically 13.56MHz (MegaHertz), signal which is supplied to the first coupling element LC1 and inductively coupled to the passive data storage device 17 via the second coupling element LC2. The voltage doubler formed by capacitor C4 and diodes D1 and D2 thus derives a power supply for the passive data storage device and, when powered, the clock deriver 600c derives a clock signal from the interrupted oscillator signal and control data is output from the data store 59 on output 59a under the control of the counter 600a.

When control data is output by the data store 59 on the output line 59a, the data switches the FET T1. The loading across the inductor L2 varies in dependence upon whether the FET T1 is conducting or non-conducting, causing the oscillating signal to be modulated in accordance with the control data output from the data store 59.

The control data output from the data store 59 is extracted from the modulated oscillating signal by the demodulator 45, modulation level detector 47, threshold detector 49 and reader microprocessor 51 to provide a data input signal to the cellular telephone processor 23 (Figures 2 and 3) representing the data output from the data store 59.

The control data output from the data store 59 and downloaded by the reader microprocessor 51 (Figure 5) to the cellular telephone processor 23 (Figure 2) may then be stored in the writable portion of the memory 29. This control data may comprise at least one of software data, that is computer code executable or implementable by the cellular telephone processor 23, and information data that is stored in the memory 29 so as to be usable by the cellular telephone processor 23. As an example of software data, the data downloaded from the passive data storage device may include upgrades or modifications of the cellular telephone processor software where the fascia is supplied by or under licence from the manufacturer or supplier of the cellular telephone. Additionally or alternatively, software data may include updates or modifications to existing games software provided with the cellular telephone or new games software.

Information data may be provided as an alternative to or in addition to software data. Examples of information data that may be stored by the data store 59 include dialling or ringing tones and telephone numbers, Internet addresses and/or WAP addresses enabling, for example, the supplier of the fascia to attract buyers by supplying new ringing tones and/or to advertise itself or other companies by supplying their telephone numbers, Internet addresses or WAP addresses. Other examples of control data may include graphics data, audio data, image data, video data, biometric data, cellular telephone services, subscription services, network services, promotions, advertisements and so on.

As described above, the control data stored by the passive data storage device consists of the actual software and/or information data or the identity or address of the user's mobile network provider or another third party, service provider. As another possibility, the control data stored by the passive data storage device may be access data that enables the user to access such software and/or information data. For example, this access data may consist of a cellular telephone number or a WAP address that the user of the cellular telephone can contact to download new software data and/or information data or the identity or address of the user's mobile network provider or another, third party, service provider. As another possibility, downloading of access data may cause the cellular telephone processor 23 to make additional facilities for which the cellular telephone processor is already programmed but that are currently barred from the user available to the user, for example facilities such as international calling access, voicemail and message-taking facilities. As a further possibility, such access data may cause the cellular telephone processor 23 to enable the user to access data previously stored in the memory 29 but not accessible to the user. Such data may include information data such as ringing tones, telephone numbers and/or WAP addresses and software data such as modifications to the processing software of the cellular telephone and/or upgrades or modifications to games software already available to the user or new games software.

The access data may be, for example, an identity code that enables the cellular telephone processor 23 to identify the particular type of fascia 3 by comparison with data stored in its memory. The cellular telephone processor 23 may then control the cellular telephone functionality and capability available to the user in accordance with the identified fascia 3.

The cellular telephone processor may be programmed so that, if it receives no control data or receives incorrect or non-understandable control data (for example if it receives the wrong access or identity code data), then the cellular telephone processor 23 may determine that the fascia is a counterfeit or unrecognised fascia and may modify or restrict the functionality of the cellular telephone. The cellular telephone processor 23 may inhibit access to all or certain optional features or functions and may continue to inhibit use of those optional features or functions until a genuine fascia is fitted to the cellular telephone. For example, if the cellular telephone processor 23 determines that the fascia is a counterfeit fascia, then the cellular telephone processor 23 may simply limit the number of functions available via the cellular telephone, for example the cellular telephone processor 23 may limit the available number of ringing or dialling tones or message taking options, for example, or may "lock" the cellular telephone so that only emergency outgoing calls may be made.

The cellular telephone processor 23 may also display a message to the user to alert them to the fact that the replacement fascia is not genuine. Where the use of a non-genuine replacement fascia is not detrimental to the functioning of the cellular telephone and does not potentially detrimentally affect the safety of the user, then the cellular telephone processor 23 may simply display a message to the user on the display 37 to alert the user to the fact that the fascia is not genuine.

The cellular telephone processor may also be programmed to enable it to identify genuine replacement fascias for other models of cellular telephone and to cause the display 37 to display to the user a message alerting them to the fact that the incorrect replacement fascia has been fitted if the cellular telephone processor determines that incorrect identity data has been received.

As described above, the passive data storage device 17 is powered and so supplies its stored data to the reader unit 15 whenever the first and second coupling elements LC1 and LC2 are inductively coupled. The reader microprocessor may send the received data continually or periodically to the cellular telephone processor 23. Continually powering the passive data storage device 17 may, however, present a drain on the battery of the cellular telephone. Accordingly, the reader microprocessor 51 may be programmed to cause the oscillator control 41 to switch on the oscillator 43 only at predetermined intervals so that the passive data storage device 17 is periodically activated to send its data. Generally, it will be desirable for the passive data storage device 17 to transmit its data to the reader unit 15 only when a replacement fascia 3 has just been coupled to the main body 5 of the cellular telephone. This may be achieved by using a simple interlock system which provides a signal to the reader microprocessor 51 indicating whether or not a fascia 3 is present. As an example, part of the encapsulating housing of the passive data storage device 17 may carry a conductive strip (17a in Figure 1) that, when the fascia 3 is coupled to the main body 5, couples a pin (51a in Figure 5) of the reader microprocessor 51 to ground or earth and the reader microprocessor 51 may periodically check the status of that pin. When the reader microprocessor 51 detects that the voltage at the pin 51a has gone high then it determines that the fascia 3 has been removed and when it detects that the voltage at that pin has again gone low it determines that either the fascia 3 has been refitted to the main body 5 or a different fascia 3 has been fitted and so activates the oscillator control 41 to switch on the power oscillator 43 to power up the passive data storage device 17. In this way, the passive data storage device 17 will be powered up to transmit its data only when a replacement fascia 3 or cover portion is fitted to the main body 5.

In the above described embodiments, the passive data storage device 17 is a synchronous passive data storage device, that is the clock signal of the passive data storage device 17 is controlled by and so synchronised with the reader microprocessor 51 However, the passive data storage device 17 may be an asynchronous device, that is the clock deriver 600c coupled to the junction J1 shown in Figure 6 may be replaced by a clock signal generator which is powered up when a power supply is derived by the passive data storage device to generate an independent clock signal for the passive data storage device. Also, although Figure 6 shows the use of a counter 600a to count out data from the data store 59 it will, of course, be appreciated that the data store 59 may be arranged simply to output its data an address at the time and that the counter 600a may not be necessary. In addition, the passive data storage device 17 may incorporate a microcontroller that controls read out of data from the data store 59 which may enable different areas of the data store 59 (and so different data) to be accessed and read out in accordance with instructions supplied by the reader microprocessor 51. Such instructions may be transmitted by representing. 0 and 1 as long and short duration interruptions of the oscillator signal.

In the above described embodiments, the passive data storage device modulates the signal received from the reader by, under control of the counter 600a and clock divider 600c, reading data out of the data store 59.

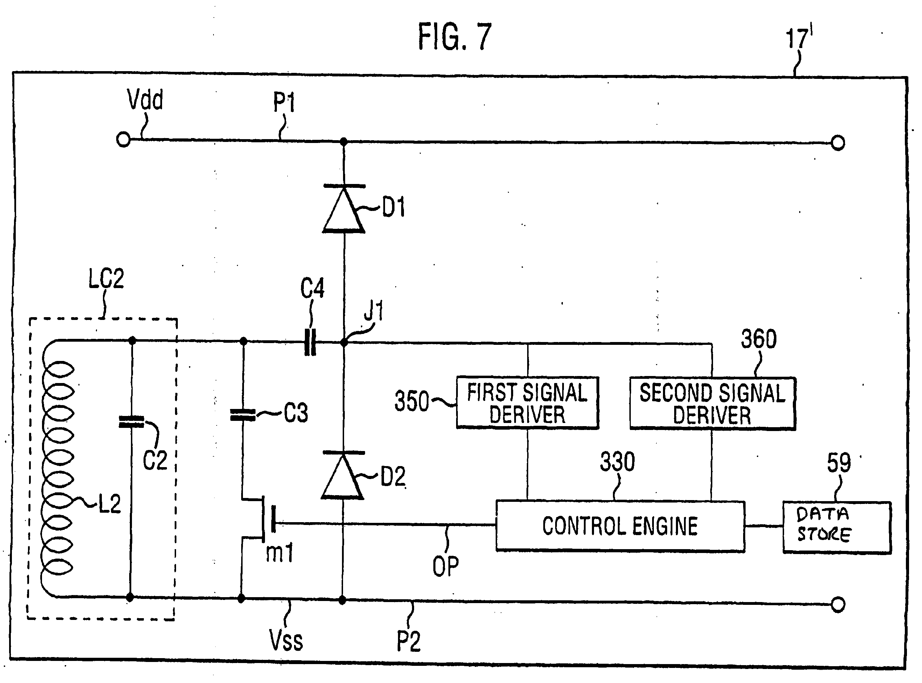

Figure 7 shows an example of another passive data storage device 17' that may be used in place of the passive data storage device 17 shown in Figure 6. This data storage device uses a different controller from that shown in Figure 6. In this case, the controller comprises a control engine 330 arranged to recognise a number of different codes (in the form of predetermined sequences of ones and zeros) transmitted to it by the reader microprocessor 51. In this example, the reader microprocessor 51 is arranged to transmit ones and zeros by causing the oscillator control 41 to interrupt the output of the oscillator 43 for short and long durations, respectively.

In this embodiment, a clock signal for the control engine 330 is derived from the signal supplied by the oscillator 43 by a first signal deriver 350 in the form of a fast missing pulse detector coupled to junction J1 while the control engine 330 is arranged to extract the data transmitted by the reader 17' using the output of the first signal deriver 350 and the output of a second signal deriver 360 in the form of a slow missing pulse detector also coupled to the junction J1.

The timeout periods of the fast and slow missing pulse detectors are set so that the output of the fast missing pulse detector will have one of two widths dependent upon whether the particular data bit is a binary "zero" or a binary "one" while the slow missing pulse detector will provide a pulse only when a particular data bit is a binary "one". The control engine 330 can thus determine from the outputs of the first and second signal derivers 350 and 360 whether a received bit is a binary "zero" or a binary "one". As set out above, the control engine 330 is programmed to enter a number of different states dependent upon the instruction code received from the reader unit 15. These different states may cause the control engine to read out data from different areas of the data store 59. The data store 59 may, however, be an electrically erasable memory in which case at least one of the states of the control engine 330 may enable the control engine to erase the data in a portion of the data store 59 and to write new data supplied by the reader unit 15 into the data store 59. This would enable, for example, the reader unit 15 to overwrite at least a part of the content of the data store 59 after the fascia 3 has first been coupled to the main body 5 so as to limit or inhibit access to the functionality provided by the data stored in the data store 59 if the fascia is subsequently removed and fitted to a different cellular telephone. As another possibility, the cellular telephone processor may be programmed to place a time limit on the availability of the additional functions provided by the data stored in the data store 59.

Although in the above described embodiment, the control engine 330 is a state machine with its own non-volatile memory it will, of course, be appreciated that the state machine may be replaced by an appropriately programmed microprocessor or microcontroller also having its own memory. Also, different methods for communicating instructions from the reader 15 to the control engine 330 may be used.

Where, as described above, the data store 59 is writable, then it may also be used to extend the memory capacity of the cellular telephone to store further telephone numbers and/or other data. The passive storage device 17 may also be used to provide a record of historical degree and frequency of use of the cellular telephone. Further details of the writing and reading operations that may be carried out by the control engine 330 are to be found in UK Patent Application number: 0031518.4 (GB-A-2370462) or the corresponding PCT application number GB01/05690 (WO02/052419). Data may also be written to the cellular telephone SIM card by the reader unit and cellular telephone processor.

In the above described embodiments, the actual components of the user interface keypads or keyboard are carried by the main body of the cellular telephone and the fascia 3 simply provides apertures through which the keypads extend.

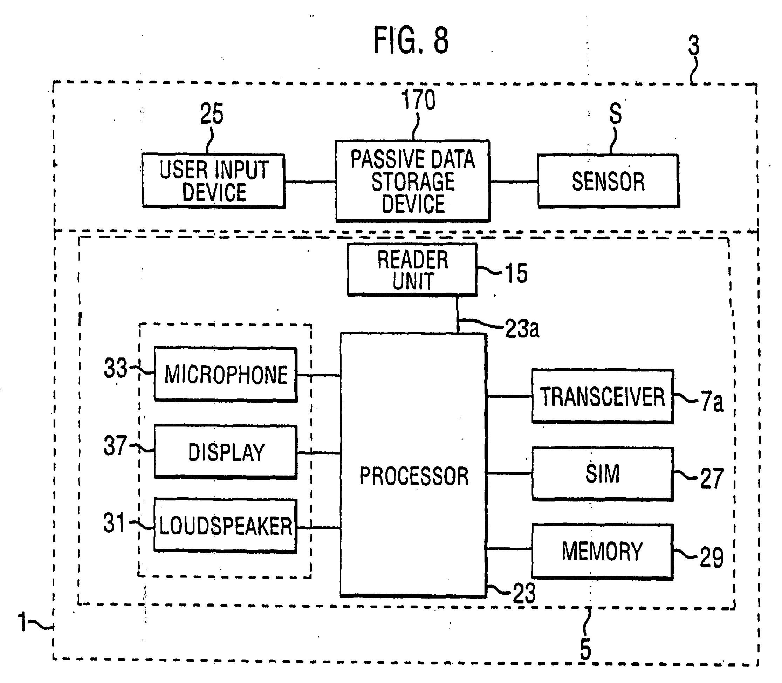

Figure 8 shows a functional block diagram, similar to Figure 2, of another example of a cellular telephone in accordance with the present invention. This cellular telephone differs from that shown in Figure 2 in that the user input device 25, in this example the keyboard, is provided within the fascia 3 and is arranged to communicate with a passive data storage device 170 that, when the fascia 3 is fitted to the main body 5 of the cellular telephone, derives a power supply from the signal supplied by the reader oscillator 43 as described above and, in response to input by the user to the user input device 25, communicates this input to the reader unit 15 by modulating the oscillator signal in a manner similar to that described above. In this case, the fascia may include an optional sensor S coupled to the passive data storage device.

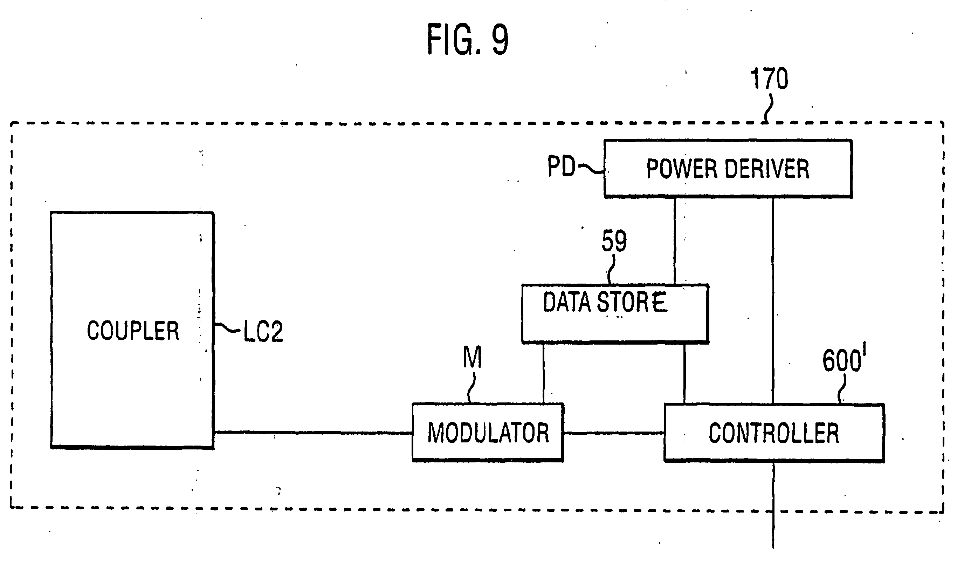

Figure 9 shows a functional block diagram of the passive device 170. This passive data storage device is similar to that shown in Figure 7 with the controller 600' having the configuration described above with reference to Figure 7 (that is it will include a control engine in the form of a state machine, microprocessor or microcontroller with its own memory). The passive date storage device 170 differs from that shown in Figure 7 in that the controller 600' incorporates a keypad or keyboard scanner (either by programming the controller or possibly by providing a dedicated user input interface) to enable it to communicate with the user input device 25 shown in Figure 8 and, in response to keystrokes made by the user of the user input device 25, to communicate to the reader unit 15 shown in Figure 8 code data (a predefined sequence of zeros and ones) that will identify to the cellular telephone microprocessor 23 the key that has been activated or depressed by the user. The passive data storage device 170 communicates this data in the same manner as described above, that is by the controller 600' causing the modulator M to modulate the oscillator signal supplied by the reader unit 15 in accordance with the data to be transmitted.

Any known form of code for identifying the keypads may be used, for example, the ASCII code system may be used. The data store 59 stores control data of the type described above, that is software data and/or information data that affects the functionality of the cellular telephone. In this case, the controller 600' of the passive data storage device 170 is programmed so as to download or communicate to the reader unit 15 control data from the data storage device 170 when the fascia is first fitted or first refitted to the main body 5 of the cellular telephone 1 and then subsequently to monitor keypad activation by the user and to communicate keystrokes input by the user to the reader unit 15 as described above.

Placing the entirety of the keyboard in the fascia 3 means that it is not necessary for the interior shell of the main body 5 of the cellular telephone to be riddled with holes corresponding to the keypad (which reduces the efficacy of any electromagnetic interference (EMI) shielding). Rather, the manufacturer of the cellular telephone should be able to use a continuous shielding layer, apart from coupling connections required to connect to the display.

Providing the entirety of the user input device 25 within the fascia 3 means that the cellular telephone may be fitted with different fascias for different applications, for example, a cellular telephone fascia that facilitates use as a cellular telephone and a games fascia that facilitates playing of a game.

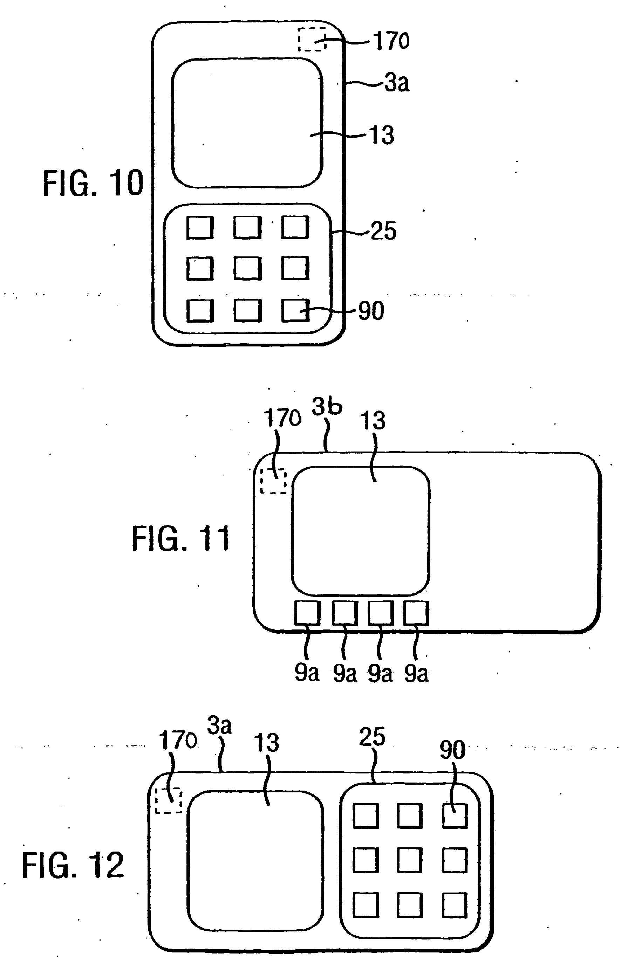

Figures 10 and 11 illustrate schematically examples of the front surfaces of two such fascias 3a and 3b. In each case, the display window 13 has the same size and dimension but in Figure 11 the display window 13 and user input device 25 are arranged so that the display is viewed in a landscape orientation while in Figure 10 the display is viewed in a portrait orientation.

The fascia 3a shown in Figure 10 has a keyboard 25 with a keypad 90 arrangement similar to that indicated by Figure 1.

In the fascia 3b shown in Figure 11, the keyboard 25 is replaced by games key inputs 9a each of which corresponds to a gaming control function, analogous to those present on a computer games console.

Separating the user input device 25 from the main body 5 of the cellular telephone enables the position, type, number and size of the keys 9a, 90 to be varied without affecting the functionality of the main body of the cellular telephone. All that is required is that the cellular telephone processor can identify from control data downloaded from the passive data storage device 170 when the fascia is first fitted, the type of user input device 25 and can then respond to the key stroke data code communicated to it from the passive data storage device 170. The control data also enables the cellular telephone processor to determine the use orientation of the display and to control the LCD accordingly.

This enables, as will be appreciated from Figures 10 and 11, the user to attach to the main body 5 a fascia carrying a user input device appropriate to the task that he wishes to carry out. Thus, as described above, a dedicated games fascia may be provided. In addition to providing a different keypad layout, the keypads themselves may be differently constructed so that, for example, soft more reactive keypads may be provided in the games fascia to allow better, faster game play. In addition, it may also be possible to build a simple joystick into the games fascia to facilitate games play.

In addition to telephone and games applications, where the cellular telephone also doubles as a personal digital assistant (PDA) having, for example, word processing and/or spreadsheet facilities, then different fascias may be provided for these options so that, for example, instead of a cellular telephone type keypad, a fascia adapted for word processing may carry, providing sufficient space is available, a full QWERTY type keyboard.

As another possibility, where the weight of the cellular telephone device or PDA is a consideration or only limited functions are required, for example whilst the user is on holiday, then a small minimalistic user input device 25 with a limited number of keypads may be provided. Also, where the user wishes to lend the cellular telephone to a younger family member, then fascias having a "restricted use" user input device may be fitted that restricts the functions of the cellular telephone that the younger family member can access.

In addition, the passive data storage device may store "macros" relating to certain keys allowing, for example, high speed input of words that relate to that particular fascia. Thus, for example, a fascia designed for use by football supporters may have words such as "goal", "net", "idiot!" and so on associated with keys of the fascia while a business fascia may have a passive data storage device that stores macros for phrases such as "call you later", "meet me at", "time", in each case facilitating, for example, the sending of text messages. In each case, because the user input device is independent of the main body or mother unit of the cellular telephone, the actual keypads may carry visual identifiers identifying the macros with which they are associated. In addition, the passive data storage device may contain as part of the control data, spell checkers, crosswords, word translators, dictionaries etc facilitating use of the cellular telephone as an educational tool.

As will be appreciated, because the user input interface device is independent of the main body 5 of the telephone, specialist keypads that enable greater levels of touch or pressure sensitivity may be provided and the controller 600' of the passive data storage device programmed to respond to different degrees of touch or pressure in a predetermined manner enabling, for example, different types of characters for example, upper, lower, bold, italics and so on, to be generated dependent on how hard the key is pressed. Such a fascia may also be specifically adapted to facilitate drawings of graphics with different keys controlling drawings of lines in different directions with the extent or thickness of the line being determined by the user pressure on the keypad or even some of the keypads being associated with simple graphical shapes such as circles, squares etc. The passive data storage device and cellular telephone processor may also be programmed to communicate data relating to musical notes and, for example, a dedicated fascia may be provided that enables the user to create tunes using different levels of pressure of touch to change the note or key, for example. Such a fascia may also be specifically adapted to enable its use by a person with a disability. For example, the fascia may be specifically adapted for use by a person having, for example a physical, visual, hearing or mental disability. As examples: a fascia may be specifically adapted for use by a visually impaired or blind person by providing the user input with specific tactile features; a fascia may be specifically adapted for use by a hearing impaired or deaf person by providing the user input with a speech-to-text facility that enables a telephone text mode; a simplified fascia may be provided with symbols and icons for people with learning difficulties; and a fascia may be provided that enables single switch operation by scanning for people with physical disabilities. As a further possibility, a fascia may provide a link to a telephone relay service such as "Typetalk" for use by hearing impaired or deaf people.

In the above described examples, separate fascias carrying the user input device are provided for different functions. As another example, the passive data storage device may contain an orientation sensor (shown as sensor S in Figures 4 and 8) that enables the controller of the passive storage device to determine the orientation of the cellular telephone and thus of the fascia so that, when the cellular telephone is in the orientation shown in Figure 10, the passive data storage device instructs the cellular telephone processor to control the display in the portrait orientation while when the cellular telephone is in the orientation shown in Figure 12, the passive data storage device instructs the cellular telephone processor to control the LCD display in landscape orientation, facilitating its use for graphics and games play. In this case, the user input device 25 itself would be physically identical in both orientations but the control signals supplied by the controller of the passive data storage device to the main body 5 of the cellular telephone in accordance with keystrokes made by the user will depend upon the orientation of the fascia.

As another possibility instead of using an orientation sensor, the fascia may carry a user input, for example one of the keypads that, when depressed, instructs the controller of the passive data storage device to communicate with the cellular telephone processor so that the orientation of the display is switched from portrait to landscape or vice versa.

The optional sensor S shown in Figures 4 and 8 need not necessarily be an orientation sensor but could be, for example, an environmental sensor sensitive to any one or more of temperature, humidity, sound, odour, a fluid, a gas, water, magnetic field, radio frequency, infra red, vibration, a chemical, pressure, for example, e.g. a barometrics sensor, orientation, position, height, or light that provides, via the first and second couplers, a signal regarding the environmental conditions to the reader unit that affects the overall operation of the cellular telephone. For example, where the sensor is a light sensor, then the reader unit may be supplied with a low light signal that automatically causes the processor of the cellular telephone to activate a back light or where the sensor senses noise level the reader unit may cause the cellular telephone device audio volume to be raised or lowered depending upon the ambient noise sensed by the sensor.

In the above described embodiments, the reader unit 15 is, as shown in Figure 2, separate from the main components of the cellular telephone. The reader unit 15 may, however, be provided within the main components of the cellular telephone. For example, the reader unit may be provided in the SIM card so that it is not necessary to adapt the reader unit for different hardware.

In the above described examples, the user input device 25 carried by the fascia is the keyboard or key input. It may, however, also be possible to incorporate into the fascia 3a or 3b within the window 13 a substantially transparent touch sensitive array such as a capacitive array that enables a user to input commands using their fingers or a stylus. In this case, the keyboard may be omitted and the display may display soft keys that are activatable by the user using the stylus or a finger. The cellular telephone processor may then be programmed to display different types of soft keys when the fascia is used in the landscape rather than the portrait orientation and, of course, different types of soft keys may be provided on different types of fascias.

In the above described examples, the user input device and passive data storage device are provided in a fascia for the cellular telephone. This need not necessarily be the case. The cellular telephone may have a conventional fascia or a fascia such as that shown in Figure 1 but without the passive data storage device and a separate user interface device having a passive data storage device and the functional components shown in Figure 9 may be provided so that, when the separate user interface device is coupled to the cellular telephone by a mechanical coupling mechanism or is positioned sufficiently close to the cellular telephone that the first and second couplers LC1 and LC2 are inductively coupled, then the controller of the passive data storage device causes control data to be read from the data storage device so as to modulate the oscillating signal to communicate to the cellular telephone instructions to the cellular telephone processor that it is to ignore the in-built user interface and to receive user input instructions from the user input device. This would enable, for example, a user to have a separate lightweight, non-self-powered user interface device having as the user interface device at least one of a keypad, keyboard, touch screen, joystick, finger print device, and digitizing tablet that enables the user, by bringing the separate user interface device into contact with or in proximity to the cellular telephone, to override the normal user interface of the telephone and to use the user interface device to facilitate, for example, playing of games software or, where the user interface device enables a QWERTY type keyboard layout, to facilitate entering of text messages or notes. It may also be possible to provide the display in the fascia.

In the above described examples, data carried by the passive data storage device affects the functionality or capabilities of the cellular telephone. Additionally, the reader processor may be programmed so as to automatically cause the cellular telephone processor to switch the cellular telephone from a standby mode to an active mode only when the fascia is correctly fitted to the main body, thereby preventing any accidental operation of the cellular telephone with its fascia removed.

Some examples of cellular telephones, in particular those incorporating PDA like facilities, include a movable, for example hinged, cover or lid. In this case, it will be appreciated that, in order for the passive data storage device to communicate with the reader unit when the cover is in the open, working condition, the passive data storage device should be appropriately located in the cover portion so as to be close to the reader unit when the cover or lid is in the open condition. As another possibility or additionally, a passive data storage device may be located in a part of the cover portion, for example, remote from a hinge, such that the couplers LC1 and LC2 are only coupled when the cover or lid is closed and the cellular telephone processor may be programmed to move from a standby to a full active mode when the reader unit provides a signal indicating that the first and second couplers LC1 and LC2 are no longer coupled.

In the above described embodiments, the passive data storage device 17 uses amplitude modulation to transmit data to the reader unit 15. It will, however, be appreciated that frequency modulation may be used as may phase modulation as described in WO 97/23060 (PCT/GB96/02975), the whole contents of which are hereby incorporated by reference.

As described above, the first and second coupling elements are arranged to couple inductively manner that requires the first and second coupling elements to be in close proximity but not necessarily in physical contact with one another This has advantages over ohmic coupling arrangements because the use of ohmic coupling has the disadvantage that actual electrical contact needs to be established between the first and second coupling elements and that this requires the coupling elements to be exposed which may make manufacture of the fascia 3 more difficult and repeated removal and fitting of fascias may cause wear and tear of the coupling elements. Also, both capacitive and ohmic coupling require more accurate alignment and closer positioning of the first and second coupling elements than inductive coupling.

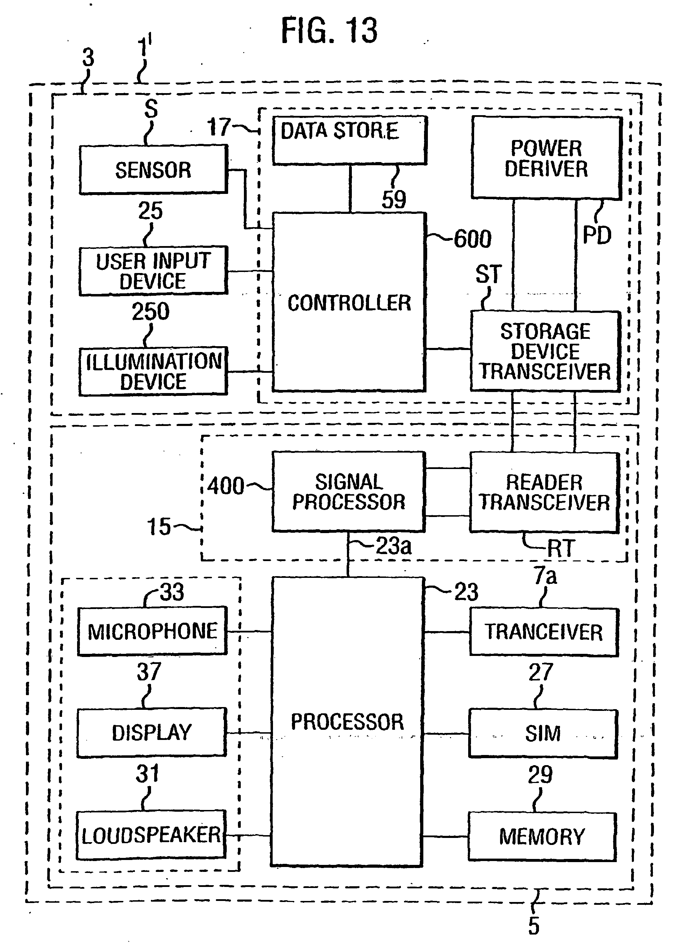

Figure 13 shows a functional block diagram of a cellular telephone 1' not falling within the scope of the invention claimed which differs from the cellular telephone described above with reference to Figures 2, 3 and 4 in a number of ways. For simplicity, the connections of the various functional components of the passive data storage device 17 (other than the controller 600) to the power deriver PD are not shown in Figure 13.

In the cellular telephone 1' shown in Figure 13 the processor 23 and memory 29 of the cellular telephone also carry out the functions of the reader processor 51 and reader memory 53 shown in Figure 3 so that these components need not be present in the reader unit 15.

In addition, derivation of power by the passive data storage device 17 and data communication between the passive data storage device 17 and the reader unit 15 is achieved by an electrical wire connection between a reader transceiver RT of the reader unit 15 and a storage device transceiver ST of the passive data storage device. The reader transceiver RT thus replaces the oscillator controller 41, oscillator 43 and coupler LC1 shown in Figure 3 while the storage device transceiver ST replaces the modulator M and coupler LC2 shown in Figure 4. The reader transceiver RT and storage device transceiver ST may communicate data by any conventional modulation technique, for example amplitude, frequency, pulse code modulation and so on.

As illustrated in Figure 13, the fascia 3 also includes an illumination device to provide a back light for the user input device 25. The illumination device 250 may be, for example, one or more, light emitting diodes (LEDs) and the controller 600 may include an LED matrix driver.

The fascia 3 may also include a sensor S as described above. The sensor S may be, for example, a sound sensor that causes the controller 600 to switch on or flash the LEDs in response to sound so enabling, for example, a visual indication of a ringing tone. As another possibility, the sensor S may be an optical input that enables direct communication with an external keypad or personal digital assistant.

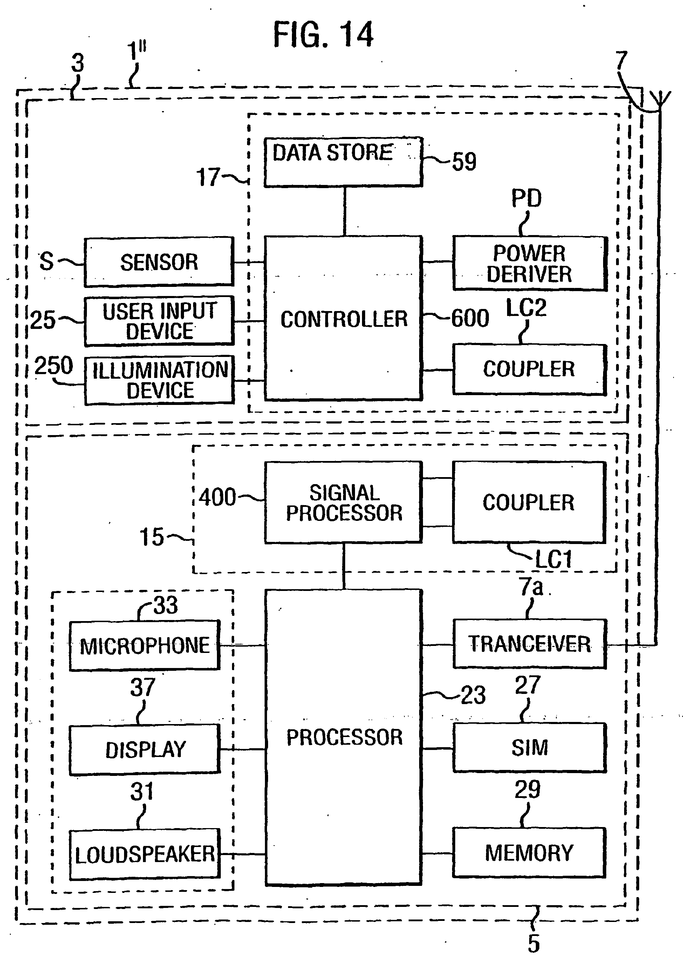

In the embodiments described above with reference to Figures 2 to 12, the reader unit 15 includes an oscillator 43 and oscillator controller 41 for generating a high frequency or RF signal from which the components carried by the fascia 3 derive a power supply and via which data communication between the passive data storage device 17 and the reader unit 15 occurs. Figure 14 shows a functional block diagram, similar to Figure 13 of another example of a cellular telephone 1" embodying the present invention wherein the oscillator and oscillator control are omitted from the reader unit and the couplers LC1 and LC2 are tuned to the RF signal emitted by the antenna 7 of the cellular telephone so that the components carried by the fascia 3 derive a power supply from the RF signal transmitted by the cellular telephone and data communication between the passive data storage device 17 and the reader unit is achieved in the manner described above with reference to Figures 6 and 7 by varying the loading of the inductive coupler LC2 to provide an amplitude modulated signal. Frequency or phase modulation may also be used, however amplitude modulation is more desirable as it should not interfere with the cellular telephone GSM signal. In other respects, the cellular telephone 1" shown in Figure 14 is similar to that shown in Figure 13.

In operation of the cellular telephone 1" shown in Figure 14, when the cellular telephone 14 is powered-up by its processor 23, the tuning of the inductive coupler LC2 to the frequency of the RF signal transmitted by the antenna 7 enables the power deriver PD to derive a power supply for the passive data storage 17 from the RF signal transmitted by the cellular telephone.

When powered-up, the passive data storage device 17 transmits data stored in the data store 59 by modulating the RF signal. The positioning of the reader unit 15 on the main body of the cellular telephone and the positioning of the passive data storage device 17 on the facia 3 are such that the coupler LC1 is positioned very close to (within 8mm of) the coupler LC2 and accordingly will pick up the modulation from the passive data storage device 17. This enables the passive data storage device to communicate its identity code to the processor 23 of the cellular telephone 1" upon powering up of the cellular telephony RF carrier signal. The coupler LC2 is, in this example, also positioned between the antenna 7 and the coupler LC1 to shadow or hide the coupler LC1 from the RF signal supplied by the antenna so that the signal supplied by the data storage device is not swamped by the antenna signal.

The processor 23 of the cellular telephone may be programmed to expect an identity code from the signal processor 400 of the reader unit 15 upon power-up so that there is no need for any handshaking operation.

The controller 600 of the passive data storage device may be programmed to transmit its identify code a number of times, for example three times, and then to cease modulation of the RF signal transmitted by the cellular telephone 1" until, after the cellular telephone has ceased transmitting, the cellular telephone next commences transmission of its RF signal.

The processor 23 of the cellular telephone may be programmed to send a clean, unmodulated, RF signal for a few milliseconds before commencing frequency modulation in accordance with the GSM standard so that the RF signal that the passive data storage device modulates is clean, that is unmodulated. This should, however, not be necessary where the passive data storage device uses amplitude modulation to convey its data to the reader unit 15.

In the above described embodiments, data is communicated between the passive data storage device 17 and the reader unit by modulating the signal from which the passive data storage device 17 derives its power.

In the example shown in Figure 13 and the embodiment shown in Figure 14, the passive data storage device remains powered while the cellular telephone is powered and a keyboard scanner included within the controller 600 receives data from the user input keypad or keyboard and communicates this to the reader unit 15. The example shown in Figure 13 and the embodiment shown in Figure 14 may, however, be modified so that the user input device (and any associated illumination device) forms part of the main body of the cellular telephone as in the embodiment described with reference to Figures 2 to 4. Incorporating the keypad or keyboard into the fascia 3 has, however, significant advantages as discussed above with reference to Figures 8 to 12.

The passive data storage device may comprise an application specific integrated circuit (ASIC) with the data store 59 comprising, for example, an electrically erasable memory, enabling the keyboard scanner software to be configured at factory level to co-operate with the particular type of keypad or keyboard carried by the particular cover into which the passive data storage device is to be incorporated.

The embodiments described with reference to Figures 2 to 12 may be modified so that the cellular telephone processor 23 carries out the functions of the reader processor 51 so that, the reader unit 15 need not include the reader processor 51 and reader memory. Conversely the example shown in Figure 13 and the embodiment shown in Figure 14 may have reader units incorporating the reader processor and memory.

The present invention may be applied to computing devices such as personal computers, PDA (personal digital assistant) or games consoles with facilities for enabling communication over a mobile telecommunications network.

The present invention may be applied to cellular telephone devices where a portion or the whole of the housing or a cover of the device is replaceable or where an attachable component such as a tool or a plug-in key or component can be attached to or fitted into or onto the device.

The present invention may be applied where the component is not necessarily attachable to the device but is an accessory that can be brought into close proximity, typically a few inches, say about or less than 10 cm, to the electrical device. For example the accessory may be a toy, a promotional item, promotional literature, an advertising hoarding or similar advertising material, a ticket, identity card, phone card, debit card or other mobile commerce product, packaging such as a product box or wrapping, vending machine or access device carrying a data storage device that contains data that affects a cellular telephone device.

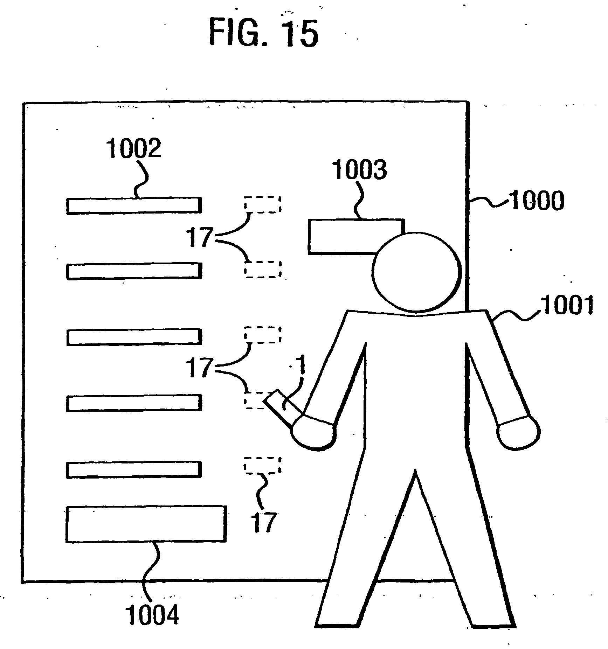

The data read by the reader unit may be used to communicate with another device, for example to enable payment for a service or product to be effected. Thus, as one example and as shown very schematically in Figure 15, a unit in the form of a product or picket vending machine 1000 may carry one or more passive data storage devices 17 each protected by an outer skin or coating of the vending machine housing that enables transmission of a radio frequency signal and so shown in dashed lines in Figure 15. Each passive data storage device 17 is associated with a corresponding box 1002 containing a brief description of the corresponding ticket or product. As shown the passive data storage devices are to the side of the boxes. They may however be within the boxes. When a user 1001 wishes to purchase a product or ticket from the vending machine he brings his cellular telephone device 1 into close proximity with the data storage device 17 associated with the desired product or ticket to enable the data storage device to derive a power supply and transmit data to the reader unit in the cellular telephone device. The reader unit in the cellular telephone device supplies the read data to the cellular telephone device processor which accesses a location on the communications network identified by the data, for example a service provider which may then debit the user's account and return an authorisation code that when entered by the user using a key pad 1003 causes the vending machine to supply the corresponding required product or ticket to an outlet 1004.

Instead of being a vending machine, the unit 1000 may be an advertising hoarding or the like with the passive data storage devices embedded in a cardboard or paper poster. In this case, the keypad and outlet will of course not be present and the user will be coupled by the cellular telephone device transceiver to source of information for example a WAP or website corresponding to a selected passive data storage device. In this case, the passive data storage device may be associated with images of different products or services, for example.

Cellular telephone devices having reader units as described above may also communicate with one another. Communication by RF or similar electromagnetic frequency has advantages over more sophisticated technology such as BlueTooth^{™} technology in that the RF communication system is cheaper and is shorter range so that there is less possibility of interference or crosstalk with other devices. Typically a reader unit will be able to communicate with a data storage device or other reader unit over a range of up to about six inches. Also, in contrast to BlueTooth^{™} which uses a frequency of about 2.4 GigaHertz, uses frequency hopping and a large number, typically 82, channels, RF communication uses a fixed single frequency, typically 13.56 MegaHertz, which provides a continuous signal which is modulated, for example amplitude modulated, by the data to be transmitted. In addition, unlike BlueTooth^{™}, such communications do not require the use of an external protocol. All that is required is that the reader unit can demodulate the demodulated carrier signal supplied by the data storage device.

Such a cellular telephone device incorporating a reader unit may be used in many forms of commerce, as a payment method, to obtain account details and so on.

## Claims

1. A cellular telephone device having :
a telecommunications radio frequency transceiver (7a) for enabling transmission and reception of communications over a mobile telecommunications network;
control means (23) for controlling operation of the cellular telephone device; and
reading means (15) having a reader (21; 23) and reader inductive coupling means (LC1) for, when the cellular telephone device is in close proximity to an article (3; 3a; 3b; 1000) carrying a data storage device (17) having a data store (59) and a data storage device inductive coupling means LC2), inductively coupling a radio frequency signal provided by a radio frequency signal supplying means of the cellular telephone device to the data storage device inductive coupling means (LC2) to cause the data storage device (17) to modulate that radio frequency signal in accordance with data stored by the data store of the data storage device,
the reading means (15) having data extracting means for extracting the data from the modulated radio frequency signal to affect an operation or service of the cellular telephone device.

2. A cellular telephone device according to claim 1, wherein the control means (23) is operable to cause transmission of a communication over the mobile telecommunications network to a location on the network determined in accordance with the data received from such a data storage device (17).

3. A cellular telephone device according to claim 1 or 2, wherein the radio frequency signal supplying means comprises the telecommunications radio frequency transceiver (7a).

4. A cellular telephone device according to claim 1 or 2, wherein the radio frequency signal supplying means is provided by the reading means (15) and is independent of the telecommunications radio frequency transceiver (7a).

5. A cellular telephone device according to claim 1, 2, or 4, wherein the reading means (15) is a reader unit consisting of the reader (21) and the reader inductive coupling means (LC1).

6. A cellular telephone device according to claim 1, 2, 3 or 4, wherein the reading means (15) comprises the control means (23).

7. A cellular telephone device according to any of the preceding claims, wherein the cellular telephone device is operable to communicate data from a memory (53) of the cellular telephone device to the article (3; 3a; 3b;1000).

8. A cellular telephone device according to claim 7, wherein the memory (53) forms part of the reading means.

9. A cellular telephone device according to claim 7, wherein the memory (53) is a read-writable memory.

10. A cellular telephone device according to any preceding claim, wherein the cellular telephone device comprises modulation means (41) operable to modulate the radio frequency signal supplied by the radio frequency signal supplying means of the cellular telephone device in accordance with data to enable, where a said data storage device (17) has a read-writable data store (59), data to be written by the cellular telephone device into the read-writable data store of the data storage device.

11. A cellular telephone device according to any of the preceding claims, wherein the reader inductive coupling means (LC1) is operable, when the cellular telephone device is in close proximity to an article (3; 3a; 3b; 1000) carrying a data storage device (17) having a data store (59) and a data storage device inductive coupling means (LC2), to couple inductively with the data storage device inductive coupling means (LC2) to cause the data storage device (17) to derive a power supply from that radio frequency signal.

12. A cellular telephone device according to any of the preceding claims, wherein the device is a cellular telephone or a computing device such as a personal computer, a personal digital assistant or a games console with facilities for enabling communication over a mobile telecommunications network.

13. A cellular telephone device according to any of the preceding claims, wherein the article comprises a removable and/or exchangeable portion (3; 3a; 3b) of the cellular telephone device.

14. A cellular telephone device according to claim 13, wherein the data storage device (17)of the article (3; 3a; 3b) has power supply deriving means (PD) for deriving a power supply from the radio frequency signal supplied by the cellular telephone device when the removable and/or exchangeable portion (3; 3a; 3b) is attached to the cellular telephone device and the reader and data storage device inductive coupling means (LC1 and LC2) are inductively coupled, data storage device control means (600) for reading data from the data store of the data storage device in response to derivation of a power supply by the power supply deriving means (PD), and modulation means (M) for modulating the radio frequency signal in accordance with the data stored by the data store (59) of the data storage device (17).

15. A data transmission system comprising a cellular telephone device according to any of the preceding claims and the article (3; 3a; 3b; 1000).

16. A data transmission system comprising a cellular telephone device according to any of claims 1 to 10 and the article (3; 3a; 3b), wherein the data storage device of the article (3; 3a; 3b) has power supply deriving means (PD) for deriving a power supply from the radio frequency signal supplied by the cellular telephone device when the reading means and data storage device inductive coupling means (LC1 and LC2) are inductively coupled when the cellular telephone device is in close proximity to the article (3; 3a; 3b), data storage device control means (600) for reading data from the data store (59) of the data storage device (17) in response to derivation of a power supply by the power supply deriving means (PD), and modulation means (M) for modulating the radio frequency signal in accordance with the data stored by the data store (59) of the data storage device (17).

17. A data transmission system according to claim 15 or 16, wherein the article comprises at least one of an accessory (3) for the cellular telephone device, a user interface (3a; 3b) for the cellular telephone device, a user interface in a fascia for the cellular telephone device, a toy, a promotional item, promotional literature, an advertising hoarding or similar advertising material, a ticket, an identity card, a phone card, a debit card or other mobile commerce product, packaging such as a product box or wrapping, a vending machine (1000) or an access control device, carrying a data storage device, or another cellular telephone device.

18. A data transmission system according to claim 15, wherein the article comprises another such cellular telephone device so that, in operation, when the cellular telephone devices are in close proximity, one of the cellular telephone devices modulates a radio frequency signal provided by the other cellular telephone device in accordance with the data stored in a data storage device (17), and the data extracting means of the other cellular telephone device extracts the data from the modulated radio frequency signal, thereby enabling communication of the data between the two cellular telephone devices.

19. A product information providing system comprising a cellular telephone device in accordance with any of claims 1 to 14, wherein different products are associated with corresponding data storage devices each of which, in response to receipt of a radio frequency signal from a cellular telephone device located in the vicinity of the data storage device, causes the cellular telephone device to communicate with an information data source relating to the product associated with that data storage device.

## Patentansprüche

1. Mobiltelefongerät mit:
einem Telekommunikations-Radiofrequenz-Transceiver (7a) für die Übertragung und den Empfang von Kommunikationen über ein Mobilfunknetz;
Steuerungsmittel (23) zum Steuern eines Betriebs des Mobiltelefongeräts; und
Lesemittel (15) mit einem Leser (21; 23) und einem Leser-Induktivkoppelmittel (LC1), um, wenn sich das Mobiltelefongerät in unmittelbarer Nähe zu einem Gegenstand (3; 3a; 3b; 1000) befindet, der ein Datenspeichergerät (17) mit einem Datenspeicher (59) und einem Datenspeichergerät-Induktivkoppelmittel (LC2) besitzt, ein Radiofrequenzsignal, das durch ein Radiofrequenzsignal-Zufuhr-Mittel des Mobiltelefongeräts bereitgestellt ist, auf das Datenspeichergerät-Induktivkoppelmittel (LC2) induktiv zu koppeln, um zu bewirken, dass das Datenspeichergerät (17) das Radiofrequenzsignal in Übereinstimmung mit Daten moduliert, die von dem Datenspeicher des Datenspeichergeräts gespeichert sind,
wobei das Lesemittel (15) ein Datenextraktionsmittel zur Extraktion der Daten vom modulierten Radiofrequenzsignal aufweist, um einen Betrieb oder einen Dienst des Mobiltelefongeräts zu beeinflussen.

2. Mobiltelefongerät nach Anspruch 1, worin das Steuerungsmittel (23) betrieben werden kann, um eine Übertragung einer Kommunikation über das Mobilfunknetz an einen Ort im Netz zu bewirken, der in Übereinstimmung mit den von solch einem Datenspeichergerät (17) empfangenen Daten ist.

3. Mobiltelefongerät nach Anspruch 1 oder 2, worin das Radiofrequenzsignal-Zufuhr-Mittel den Telekommunikations-Radiofrequenz-Transceiver (7a) umfasst.

4. Mobiltelefongerät nach Anspruch 1 oder 2, worin das Radiofrequenzsignal-Zufuhr-Mittel durch das Lesemittel (15) bereitgestellt ist und vom Telekommunikations-Radiofrequenz-Transceiver (7a) unabhängig ist.

5. Mobiltelefongerät nach Anspruch 1, 2 oder 4, worin das Lesemittel (15) eine Leseeinheit ist, die aus dem Leser (21) und dem Leser-Induktivkoppelmittel (LC1) besteht.

6. Mobiltelefongerät nach Anspruch 1, 2, 3 oder 4, worin das Lesemittel (15) das Steuerungsmittel (23) umfasst.

7. Mobiltelefongerät nach einem der voranstehenden Ansprüche, worin das Mobiltelefongerät betrieben werden kann, um Daten von einem Internspeicher (53) des Mobiltelefongeräts zum Gegenstand (3; 3a; 3b; 1000) zu kommunizieren.

8. Mobiltelefongerät nach Anspruch 7, worin der Internspeicher (53) einen Teil des Lesemittels bildet.

9. Mobiltelefongerät nach Anspruch 7, worin der Internspeicher (53) ein Schreib-Lese-Internspeicher ist.

10. Mobiltelefongerät nach einem der voranstehenden Ansprüche, worin das Mobiltelefongerät ein Modulationsmittel (41) umfasst, das betrieben werden kann, um das von dem Radiofrequenzsignal-Zufuhr-Mittel des Mobiltelefongeräts zugeführte Radiofrequenzsignal in Übereinstimmung mit Daten zu modulieren, um es zu ermöglichen, wo ein Datenspeichergerät (17) einen Schreib-Lese-Speicher (59) aufweist, dass vom Mobiltelefongerät Daten in den Schreib-Lese-Speicher des Datenspeichergeräts hinein geschrieben werden.

11. Mobiltelefongerät nach einem der voranstehenden Ansprüche, worin das Leser-Induktivkoppelmittel (LC1) betrieben werden kann, um, wenn sich das Mobiltelefongerät in unmittelbarer Nähe zu einem Gegenstand (3; 3a; 3b; 1000) befindet, der ein Datenspeichergerät (17) mit einem Datenspeicher (59) und einem Datenspeichergerät-Induktivkoppelmittel (LC2) besitzt, mit dem Datenspeichergerät-Induktivkoppelmittel (LC2) induktiv zu koppeln, um zu bewirken, dass das Datenspeichergerät (17) eine Energieversorgung vom Radiofrequenzsignal ableitet.

12. Mobiltelefongerät nach einem der voranstehenden Ansprüche, worin das Gerät ein Mobiltelefon oder ein Computergerät, wie beispielsweise ein Personal Computer, ein Personal Digital Assistent oder eine Spielkonsole mit Systemeinrichtungen zur Ermöglichung von Kommunikation über ein Mobilfunknetz ist.

13. Mobiltelefongerät nach einem der voranstehenden Ansprüche, worin der Gegenstand einen entfernbaren und/oder austauschbaren Bereich (3; 3a; 3b) des Mobiltelefongeräts umfasst.

14. Mobiltelefongerät nach Anspruch 13, worin das Datenspeichergerät (17) des Gegenstands (3; 3a; 3b) ein Energieversorgungs-Ableitungs-Mittel (PD) zum Ableiten einer Energieversorgung von dem durch das Mobiltelefongerät zugeführte Radiofrequenzsignal, wenn der entfernbare und/oder austauschbare Bereich (3; 3a; 3b) an dem Mobiltelefongerät angebracht ist und das Leser- und Datenspeichergerät-Induktivkoppelmittel (LC1 und LC2) induktiv gekoppelt sind, ein Datenspeichergerät-Steuerungsmittel (600) zum Lesen von Daten vom Datenspeicher des Datenspeichergeräts in Antwort auf eine Ableitung einer Energieversorgung durch das Energieversorgungs-Ableitungs-Mittel (PD), sowie Modulationsmittel (M) zum Modulieren des Radiofrequenzsignals in Übereinstimmung mit den Daten, die vom Datenspeicher (59) des Datenspeichergeräts (17) gespeichert sind, aufweist.

15. Datenübertragungssystem, das ein Mobiltelefongerät nach einem der voranstehenden Ansprüche und den Gegenstand (3; 3a; 3b; 1000) umfasst.

16. Datenübertragungssystem, das ein Mobiltelefongerät nach einem der Ansprüche 1 bis 10 und den Gegenstand (3; 3a; 3b) umfasst, worin das Datenspeichergerät des Gegenstands (3; 3a; 3b) ein Energieversorgungs-Ableitungs-Mittel (PD) zum Ableiten einer Energieversorgung von dem durch das Mobiltelefongerät zugeführte Radiofrequenzsignal, wenn das Lesemittel- und Datenspeichergerät-Induktivkoppelmittel (LC1 und LC2) induktiv gekoppelt sind, wenn sich das Mobiltelefongerät in unmittelbarer Nähe zum Gegenstand (3; 3a; 3b) befindet, ein Datenspeichergerät-Steuerungsmittel (600) zum Lesen von Daten von dem Datenspeicher (59) des Datenspeichergeräts (17) in Antwort auf Ableitung einer Energieversorgung durch das Energieversorgungs-Ableitungs-Mittel (PD), sowie Modulationsmittel (M) zum Modulieren des Radiofrequenzsignals in Übereinstimmung mit den Daten, die vom Datenspeicher (59) des Datenspeichergeräts (17) gespeichert sind, aufweist.

17. Datenübertragungssystem nach Anspruch 15 oder 16, worin der Gegenstand wenigstens eines von einem Zubehörteil (3) des Mobiltelefongeräts, eine Benutzerschnittstelle (3a; 3b) des Mobiltelefongeräts, eine Benutzerschnittstelle in einer Blende des Mobiltelefongeräts, ein Spielzeug, einen Werbeartikel, Werbeliteratur, eine reklamebezogene Anzeigentafel oder ähnliches reklamebezogenes Material, ein Ticket, einen Ausweis, eine Telefonkarte, eine Debitkarte oder anderes mobiles Handelsprodukt, Verpackung wie beispielsweise einen Warenkarton oder -verpackung, einen Verkaufsautomaten (1000) oder ein Zugangskontrollgerät, Tragen eines Datenspeichergeräts oder ein anderes Mobiltelefongerät umfasst.

18. Datenübertragungssystem nach Anspruch 15, worin der Gegenstand ein anderes Mobiltelefongerät derart umfasst, dass, bei Betrieb, wenn die Mobiltelefongeräte sich in unmittelbarer Nähe befinden, eines der Mobiltelefongeräte ein von dem anderen Mobiltelefongerät bereitgestelltes Radiofrequenzsignal in Übereinstimmung mit den Daten, die in einem Datenspeichergerät (17) gespeichert sind, moduliert, und das Daten-Extraktions-Mittel des anderen Mobiltelefongeräts die Daten von dem modulierten Radiofrequenzsignal extrahiert, um **dadurch** eine Kommunikation der Daten zwischen den zwei Mobiltelefongeräten zu ermöglichen.

19. Produktinformations-Bereitstellungs-System, umfassend ein Mobiltelefongerät in Übereinstimmung mit einem der Ansprüche 1 bis 14, worin unterschiedliche Produkte mit entsprechenden Datenspeichergeräten verbunden sind, von denen jedes, in Antwort auf Empfang eines Radiofrequenzsignals von einem in der nahen Umgebung des Datenspeichergeräts angeordneten Mobiltelefongerät, bewirkt, dass das Mobiltelefongerät mit einer Informationsdatenquelle kommuniziert, die sich auf das Produkt bezieht, das mit dem Datenspeichergerät verbunden ist.

## Revendications

1. Dispositif téléphonique cellulaire possédant :
un émetteur-récepteur de télécommunications à fréquences radio (7a), destiné à permettre l'émission et la réception de communications sur un réseau de télécommunications mobiles ;
un moyen de commande (23), destiné à commander le fonctionnement du dispositif téléphonique cellulaire ; et
un moyen de lecture (15), possédant un lecteur (21 ; 23) et un moyen de couplage inductif du lecteur (LC1), destiné, lorsque le dispositif téléphonique cellulaire est à proximité étroite d'un article (3 ; 3a ; 3b ; 1000) portant un dispositif de stockage de données (17) possédant une mémoire de données (59) et un moyen de couplage inductif du dispositif de stockage de données (LC2), à coupler inductivement un signal de fréquence radio, fourni par un moyen de fourniture de signal de fréquence radio du dispositif téléphonique cellulaire, avec le moyen de couplage inductif du dispositif de stockage de données (LC2) afin d'amener le dispositif de stockage de données (17) à moduler ce signal de fréquence radio en fonction des données stockées dans la mémoire de données du dispositif de stockage de données,
le moyen de lecture (15) possédant un moyen d'extraction de données destiné à extraire les données du signal de fréquences radio modulé afin d'influer sur le fonctionnement ou le service du dispositif téléphonique cellulaire.

2. Dispositif téléphonique cellulaire selon la revendication 1, dans lequel le moyen de commande (23) est en mesure de commander l'établissement d'une communication sur le réseau de télécommunications mobiles, vers un lieu sur le réseau qui est déterminé en fonction des données reçues d'un tel dispositif de stockage de données (17).

3. Dispositif téléphonique cellulaire selon la revendication 1 ou 2, dans lequel le moyen de fourniture de signal de fréquence radio comprend l'émetteur-récepteur de télécommunications à fréquences radio (7a).

4. Dispositif téléphonique cellulaire selon la revendication 1 ou 2, dans lequel le moyen de fourniture de signal de fréquence radio est fourni par le moyen de lecture (15) et est indépendant de l'émetteur-récepteur de télécommunications à fréquences radio (7a).

5. Dispositif téléphonique cellulaire selon la revendication 1, 2 ou 4, dans lequel le moyen de lecture (15) est une unité de lecture constituée du lecteur (21) et du moyen de couplage inductif du lecteur (LC1).

6. Dispositif téléphonique cellulaire selon la revendication 1, 2, 3 ou 4, dans lequel le moyen de lecture (15) comprend le moyen de commande (23).

7. Dispositif téléphonique cellulaire selon l'une quelconque des revendications précédentes, dans lequel le dispositif téléphonique cellulaire est en mesure de communiquer des données entre une mémoire (53) du dispositif téléphonique cellulaire et l'article (3 ; 3a ; 3b ; 1000).

8. Dispositif téléphonique cellulaire selon la revendication 7, dans lequel la mémoire (53) fait partie du moyen de lecture.

9. Dispositif téléphonique cellulaire selon la revendication 7, dans lequel la mémoire (53) est une mémoire autorisant la lecture et l'écriture.

10. Dispositif téléphonique cellulaire selon l'une quelconque des revendications précédentes, dans lequel le dispositif téléphonique cellulaire comprend un moyen de modulation (41) en mesure de moduler le signal de fréquence radio fourni par le moyen de fourniture de signal de fréquence radio du dispositif téléphonique cellulaire en fonction de données afin, lorsque ledit dispositif de stockage de données (17) possède une mémoire de données (59) autorisant la lecture et l'écriture, de faire écrire des données par le dispositif téléphonique cellulaire dans la mémoire de données autorisant la lecture et l'écriture du dispositif de stockage de données.

11. Dispositif téléphonique cellulaire selon l'une quelconque des revendications précédentes, dans lequel le moyen de couplage inductif du lecteur (LC1) est en mesure, lorsque le dispositif téléphonique cellulaire est à proximité étroite d'un article (3 ; 3a ; 3b ; 1000) portant un dispositif de stockage de données (17) possédant une mémoire de données (59) et un moyen de couplage inductif du dispositif de stockage de données (LC2), de réaliser un couplage inductif avec le moyen de couplage inductif du dispositif de stockage de données (LC2) pour amener le dispositif de stockage de données (17) à dériver une alimentation électrique de ce signal de fréquence radio.

12. Dispositif téléphonique cellulaire selon l'une quelconque des revendications précédentes, dans lequel le dispositif est un téléphone cellulaire ou un dispositif de calcul tel qu'un ordinateur personnel, un assistant numérique personnel ou une console de jeu disposant de fonctions permettant une communication sur un réseau de télécommunications mobiles.

13. Dispositif téléphonique cellulaire selon l'une quelconque des revendications précédentes, dans lequel l'article comporte une partie amovible et/ou interchangeable (3 ; 3a ; 3b) du dispositif téléphonique cellulaire.

14. Dispositif téléphonique cellulaire selon la revendication 13, dans lequel le dispositif de stockage de données (17) de l'article (3 ; 3a ; 3b) possède un moyen de dérivation d'alimentation électrique (PD), destiné à dériver une alimentation électrique du signal de fréquence radio fourni par le dispositif téléphonique cellulaire lorsque la partie amovible et/ou interchangeable (3 ; 3a ; 3b) est rattachée au dispositif téléphonique cellulaire et que les moyens de couplage inductif du lecteur et du dispositif de stockage de données (LC1 et LC2) sont couplés inductivement, possède un moyen de commande de dispositif de stockage de données (600) destiné à lire des données dans la mémoire de données du dispositif de stockage de données en réponse à la dérivation d'une alimentation électrique par le moyen de dérivation d'alimentation électrique (PD) et possède un moyen de modulation (M), destiné à moduler le signal de fréquence radio en fonction des données stockées dans la mémoire de données (59) du dispositif de stockage de données (17).

15. Système de transmission de données comprenant un dispositif téléphonique cellulaire selon l'une quelconque des revendications précédentes et l'article (3 ; 3a ; 3b ; 1000).

16. Système de transmission de données comprenant un dispositif téléphonique cellulaire selon l'une quelconque des revendications 1 à 10 et l'article (3 ; 3a ; 3b), dans lequel le dispositif de stockage de données de l'article (3 ; 3a ; 3b) possède un moyen de dérivation d'alimentation électrique (PD), destiné à dériver une alimentation électrique du signal de fréquence radio fourni par le dispositif téléphonique cellulaire lorsque les moyens de couplage inductif du lecteur et du dispositif de stockage de données (LC1 et LC2) sont couplés inductivement, lorsque le dispositif téléphonique cellulaire est à proximité étroite de l'article (3 ; 3a ; 3b), possède un moyen de commande de dispositif de stockage de données (600) destiné à lire des données dans la mémoire de données du dispositif de stockage de données (17) en réponse à la dérivation d'une alimentation électrique par le moyen de dérivation d'alimentation électrique (PD) et possède un moyen de modulation (M), destiné à moduler le signal de fréquence radio en fonction des données stockées dans la mémoire de données (59) du dispositif de stockage de données (17).

17. Système de transmission de données selon la revendication 15 ou 16, dans lequel l'article comprend au moins un élément parmi les suivants : un accessoire (3) pour le dispositif téléphonique cellulaire, une interface utilisateur (3a ; 3b) destinée au dispositif téléphonique cellulaire, une interface utilisateur dans une façade destinée au dispositif téléphonique cellulaire, un jouet, un article promotionnel, de la littérature promotionnelle, un panneau publicitaire ou un autre matériel publicitaire similaire, un ticket, une carte d'identité, une carte téléphonique, une carte de débit ou un autre produit commercial mobile, un emballage tel qu'une boîte ou une enveloppe de produit, une machine de vente (1000) ou un dispositif de contrôle d'accès, portant un dispositif de stockage de données, ou un autre dispositif téléphonique cellulaire.

18. Système de transmission de données selon la revendication 15, dans lequel l'article est constitué d'un autre dispositif téléphonique cellulaire similaire, si bien que, en service, lorsque les deux dispositifs téléphoniques cellulaires sont à proximité étroite, l'un des dispositifs téléphoniques cellulaires module un signal de fréquence radio fourni par l'autre dispositif téléphonique cellulaire en fonction des données stockées dans un dispositif de stockage de données (17) et le moyen d'extraction de données de l'autre dispositif téléphonique cellulaire extrait les données du signal de fréquence radio modulé, ce qui permet la communication des données entre les deux dispositifs téléphoniques cellulaires.

19. Système de fourniture d'informations sur les produits, comprenant un dispositif téléphonique cellulaire selon l'une quelconque des revendications 1 à 14, dans lequel différents produits sont associés à des dispositifs de stockage de données correspondants dont chacun, en réponse à la réception d'un signal de fréquence radio provenant d'un dispositif téléphonique cellulaire situé à proximité du dispositif de stockage de données, fait communiquer le dispositif téléphonique cellulaire avec une source de données d'information intéressant le produit et associée à ce dispositif de stockage de données.
